(19) **Europäisches Patentamt**
**European Patent Office**
**Office européen des brevets**

(11) Publication number: **0 283 526 B1**

## EUROPEAN PATENT SPECIFICATION

(45) Date of publication of patent specification: **27.02.91**

(51) Int. Cl.⁵: **A61B 17/12, A61M 25/02**

(21) Application number: **87104040.8**

(22) Date of filing: **19.03.87**

(54) Clip apparatus.

(43) Date of publication of application:
**28.09.88 Bulletin 88/39**

(45) Publication of the grant of the patent:
**27.02.91 Bulletin 91/09**

(84) Designated Contracting States:
**BE CH DE ES FR GB GR IT LI NL SE**

(56) References cited:
**DE-A- 1 603 949      DE-A- 2 730 691**
**US-A- 2 729 876      US-A- 3 068 870**
**US-A- 3 302 648      US-A- 3 802 437**
**US-A- 3 971 270      US-A- 4 398 907**
**US-A- 4 484 911**

(73) Proprietor: **Tretbar, Lawrence L.**
**Georgetown Medical Building 8901 West
74th Street Suite 300
Shawnee Mission, Kansas 66204(US)**

(72) Inventor: **Tretbar, Lawrence L.**
**Georgetown Medical Building 8901 West
74th Street Suite 300
Shawnee Mission, Kansas 66204(US)**

(74) Representative: **Baillie, Iain Cameron
c/o Ladas & Parry Isartorplatz 5
D-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

Background of the Invention

This invention relates to a medical clip and, in particular, to a clip apparatus utilized in cholangiography and similar procedures wherein a catheter or the like is placed in a duct or vessel and held therein by a clamp mechanism. It also relates to a tool for applying and removing such a medical clip.

Many medical procedures require that a catheter, tube or the like be placed in a body duct or vessel and held therein while some medical procedure is performed. For example, plastic catheters are often placed in veins and held therein by a stitch or by a loop of string tied around the catheter. Similarly, a tubular structure must be held within a vessel during a lymphangiogram or in an IV cut down procedure. During cholangiography, a cannula must be sealably held within the cystic duct.

In humans, the common bile duct joins the liver with the duodenum to provide flow of bile from the liver to the small intestine to assist in the digestion of fats. The gallbladder is a sac-like structure which communicates with the common bile duct through the cystic duct and provides intermittent storage for excess bile produced by the liver, especially between meals. Gallstones sometimes occur in the bile and block or impede flow of the bile through the various ducts. When this occurs, the gallbladder may have to be surgically removed (cholecystectomy).

To ensure removal of all gallstones from the common bile duct, cholangiography is normally perfomed on the ductile system of the biliary tract which comprises the injection of a radiopaque dye into the duct followed by a series of x-rays. The x-rays tend to image the outlines of any stones or obstructions within the ducts, as variances in opacity occur where the stones prevent the dye from filling the duct.

Normally, the dye is injected into the common bile duct through a relatively small catheter which is inserted into a severed end of the cystic duct. It is important to form a relatively tight fluid seal between the cystic duct and the catheter. Such a seal prevents the dye from leaking out of the cystic duct rather than passing through the common bile duct to image the gallstones therein.

In order to produce such a tight seal, surgeons initially utilized a suture which was tied securely around the cystic duct to hold the catheter in place. Because it is difficult and cumbersome to tie the suture at this location, many surgeons have elected to utilize a commercially available metallic hemoclip for this purpose. The hemoclips were designed to have opposing walls which flatten against one another to flatten a severed blood vessel or the like to prevent leakage therefrom. As this type of clip was designed to compress flatly and not in a circle, there is a tendency for the tissue of the cystic duct to ovate when the clip is applied so that leakage occurs between the cystic duct and the catheter on the sides of the duct not compressed by the clip. In addition, the clip must be pried loose to remove.

Some complex mechanical clips have also been designed to be used for this purpose, see e.g. US-A-4484911. However, such clips tend to be cumbersome as well as relatively expensive.

Hence, it was desired to produce a relatively simple clip which could be easily and quickly applied to the cystic duct which would compress the cystic duct fairly evenly about its entire circumference and which would be fairly easily removed after completion of the cholangiograpic process.

Further, it was desired to provide a tool which would both readily apply and remove the clip.

Objects of the Invention

Therefore, the objects of the present invention are:
to provide a medical clip providing a relatively fluid-tight seal between a vessel and a tube within the vessel when the clip is cooperatively placed around the vessel; to provide such a clip which compresses the vessel relatively evenly about the entire circumference of the vessel; to provide such a clip which is relatively easy to apply and simple in nature; to provide such a clip which is relatively easy to remove; to provide such a clip which is especially well adapted for use in compression of the cystic duct about a catheter during cholangiography; to provide such a clip which includes a pair of opposed C-shaped jaws interconnected by arms at a semimaleable juncture which allows selective positioning of the jaws when pressure is applied to the arms; to provide such a clip wherein the arms may be partially urged toward one another to compress the jaws and wherein the arms can be further compressed in such a manner as to urge the jaws apart to facilitate removal of the clips; to provide such a clip so that it is usable with catheter having serrations thereon for cooperating with the clip to form a fluid-tight seal between the catheter and the duct; to provide an application and removal tool for the clip; to provide such a clip which is relatively simple and inexpensive to make, simple to use and particularly well adapted for the intended usage thereof.

U.S. Patent 3,068,870 shows a U-shaped clip applied by a tong or plier-like applicator. The applicator serves only to crimp and there is no suggestion of a fulcrum arrangement which can release an applied clip.

U.S. Patent 4,398,907 discloses a "figure-eight" clamping device requiring a spring to retain the device in the operative position. The clamping device has arms and jaws with a hinged interconnection. Again, there is no fulcrum arrangement.

According to a first aspect of the invention, there is provided a medical clip for removably clamping a living vessel about a tube during a medical procedure so as to hold the tube within the vessel, said clip comprising a pair of opposed jaws and a pair of arms connected at one of their ends to the jaws, characterized in that the arms are joined at their other end by a maleable hinge which allows the angular spacing between the arms to be varied by application of pressure to the arms, and in that at least one of the arms is bent medially therealong outwardly with respect to the other of arms, the ends of the jaws in touching relationship are adapted to function as a fulcrum so that, when further pressure is applied to said arm, the respective jaw pivots about said fulcrum, and the jaw ends opposite said fulcrum become spaced apart so as to allow release of the clip from the vessel. According to a second aspect of the invention a tool is provided which is adapted for application and removal of the inventive clips.

In the accompanying drawings:

Fig. 1 is a fragmentary view of internal organs of a human including liver, gallbladder, common bile duct and duodenum shortly prior to severing of the cystic duct connecting the gallbladder with the common bile duct with a surgical instrument.

Fig. 2 shows insertion of a cholangiographic catheter into the cystic duct after the gallbladder is severed therefrom.

Fig. 3 is a side elevational view of a clip according to the present invention.

Fig. 4 is a side elevational view of the clip in a closed position just subsequent to applying the clip to the cystic duct by utilization of a clip applying tool.

Fig. 5 is an enlarged and fragmentary perspective view of the common bile duct and cystic duct with the catheter placed within the cystic duct and held therein by the clip.

Fig. 6 is a fragmentary and enlarged cross-sectional view of the common bile duct and cystic duct with the catheter positioned within the cystic duct and held therein by the clip, taken along line 6-6 of Fig. 5.

Fig. 7 is a fragmentary and enlarged side elevational view of the common bile duct and cystic duct showing the clip in an open position after removal from the cystic duct and showing the clip applying tool being utilized to remove the clip.

Fig. 8 is a perspective view on a smaller scale of the clip applying tool.

## Detailed Description of the Invention

As required, detailed embodiments of the present invention are disclosed herein; however, it is to be understood that the disclosed embodiments are merely exemplary of the invention which may be embodied in various forms.

The reference numeral 1 generally designates a clip in accordance with the present invention. The clip l comprises a pair of opposed C-shaped jaws 3 and 4 interconnected by a handle 5. Although the clip l is shown utilized in conjunction with a cholangiographic process as described below, it is foreseen that the clip l may be utilized in other medical procedures requiring the sealing of a vessel or duct about a circular tube or the like.

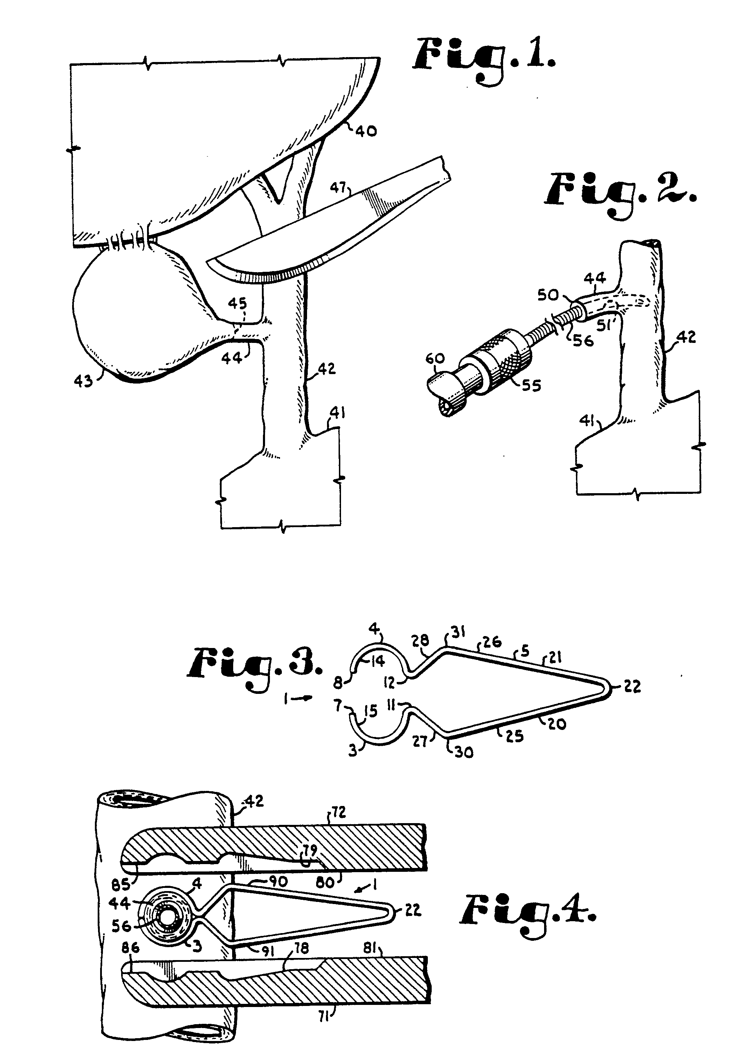

As seen in Fig. 3, the jaws 3 and 4 are spaced from one another prior to use. The jaws 3 and 4 each have distal ends 7 and 8 respectively which are spaced opposite one another. The jaws 3 and 4 also have fulcrum ends ll and l2 respectively which are also spaced opposite one another prior to use of the clip l. The ends ll and l2 are directly attached to the handle 5. The jaws 3 and 4 have facing surfaces l4 and l5 which are generally semicircular in cross-section and concave. In certain procedures where the clip is to be positioned on the vessel for a relatively long time, the jaw surface may be slightly parabolic rather than semicircular to allow blood to flow from one side of the clip to the other.

The handle 5 includes arms 20 and 21 joined at a maleable but not springy hinge 22. The hinge 22 and, preferably, the entire clip l is constructed of a generally nonresiliant but maleable material which may be bent or manipulated into a modified position when pressure or force is applied thereto, but which substantially retains the modified position when the force is removed. Therefore, if force is applied to the arms 20 and 21 to swing the arms relative to one another about the hinge 22 and change the angle thereof with respect to one another, the arms 20 and 21 will retain the new angular relationship between each other after the pressure is removed.

Each of the arms 20 and 21 include a first portion 25 and 26 and a second portion 27 and 28 respectively. The arm first portions 25 and 26 are relatively straight and join at the hinge 22. The arm second portions 27 and 28 are also relativley straight and join at one end thereof with the arm first portions 25 and 26 respectively opposite the hinge 22 and at the other end thereof with the jaw fulcrum ends ll and l2 respectively.

Prior to use, the arms 20 and 2l are not linear between the jaw fulcrum ends ll and l2 respectively and the hinge 22, but rather bow outwardly from one another. In particular, the arm portions 25 and 27 meet at juncture 30 and the arm portions 26 and 28 meet at juncture 3l such that junctures 30 and 3l are spaced substantially outward from linear projections between the hinge 22 and the jaw fulcrum ends ll and l2 respectively.

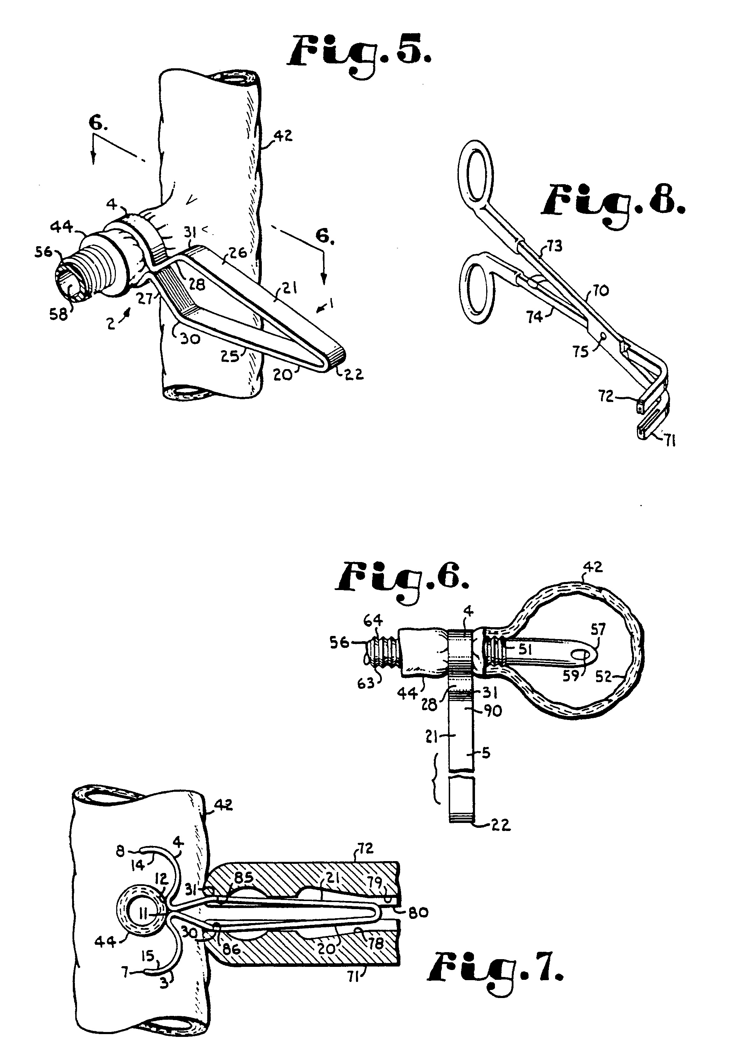

The clip l has a first configuration which is shown in Fig. 3 and corresponds to the clip l prior to use thereof. The clip l has a second configuration shown in Fig. 4 which corresponds to the configuration of the clip l during use thereof. The clip l has a third configuration which is shown in Fig. 7 and which corresponds to the configuration of the clip l subsequent to removal thereof following use. These three configurations will be discussed in greater detail below.

Fig. l shows part of the internal organs of a human. In particular, a liver 40 is shown interconnected with a duodenum 4l by a common bile duct 42. The bile duct 42 is further connected to a gallbladder 43 by a cystic duct 44. The gallbladder 43 is about to be severed from the cystic duct 44 along an imaginary cut line 45 by a surgical instrument 47. Fig. 2 shows the common bile duct 42 and cystic duct 44 subsequent to severing of the gallbladder 43 from the cystic duct 44. The cystic duct has a free and open end 50 communicating with an internal lumen 5l thereof. The common bile duct 42 also has an internal lumen 52, as can be seen in Fig. 6.

Also in Fig. 2, there is shown an instrument 55 for injecting a radiopaque dye into the common bile duct 42. The instrument 55 comprises a catheter 56 having a tip 57, an internal lumen 58 and an aperture 59 near the tip 57 opening into the lumen 58. The instrument 55 further includes a syringe 60 connected to the catheter 56 by a Luer Lock or the like and partially seen in Fig. 2. The syringe 60 holds dye and allows an operator to urge dye into and through the catheter 56. The catheter 56 is corrugated so as to have a plurality of ridges or serrations 63 generally equally spaced along a substantial portion of an outer surface 64 thereof.

As seen in Fig. 8 and partially in Figs. 4 and 7, a tool 70 is employed having opposed jaws 7l and 72, arms 73 and 74 joined with said jaws 7l and 72 respectively and a hinge or pivotal connection 75 positioned medially along the arms 73 and 74. The arms 73 and 74 are fixedly attached to the jaws 7l and 72 respectively such that the jaws 7l and 72 extend at right angles to the arms 73 and 74. The arms 73 and 74 are joined by the pivotal connection so as to form a pliers-like tool wherein movement of the distal ends of the arms 73 and 74, opposite the jaws 7l and 72, together urges the jaws 7l and 72 together whereas outward movement of the distal ends of the arms 73 and 74 relative to one another separates or spaces the jaws 7l and 72.

The tool jaws 72 and 73 have recesses 78 and 79 respectively positioned on facing surfaces 80 and 8l thereof. The recesses 78 and 79 are shaped to roughly conform with outer surfaces 90 and 9l of a nonused clip l.

In Fig. 4, the jaws 7l and 72 are spaced from the clip l, but positioned such that the recesses 78 and 79 are generally aligned to mate with the outer surfaces 90 and 9l of the clip l. The tool jaws 7l and 72, as shown in Fig. 4, have just released the clip l after compressing the C-shaped jaws 3 and 4 of the clip l together so that jaw distal ends 7 and 8 touch and jaw fulcrum ends ll and l2 touch. During such compression, the clip arm first portion 25 and second portion 27 as well as the clip arm first portion 26 and second portion 28 remain substantially unchanged and generally retain the same angle therebetween. Each of the jaws 7l and 72 have a face forward portion 85 and 86 respectively which are generally parallel and do not align with any particular structure of the clip l when the clip l is being installed by the tool 70.

The surfaces 85 and 86 however can be aligned to be positioned in opposed relationship to each other over the junctures 30 and 3l of the arms 20 and 2l respectively, as seen in Fig. 7. Upon movement of the jaws 7l and 72 together, the junctures 30 and 3l, which are V-shaped, are pressed toward each other so as to make a wider V, that is, to change the relative angle between the first portions 25 and 26 and second portions 27 and 28 of the arms 20 and 2l respectively so as to make the arms 20 and 2l closer to being linear. Such modifications of the arms 20 and 2l rotate the clip jaws 3 and 4 about their fulcrum ends ll and l2 so that the jaw distal ends 7 and 8 substantially separate, as is shown in Fig. 7. The clip l is then in the open configuration thereof and easily removed from the cystic duct 44.

It is also foreseen that only one of the arms of the clip could be bent in order for the clip to open as described above; however, only one of the jaws would rotate about the fulcrum point.

Therefore, in use, the clip l is illustrated in conjunction with a cholangiography process. In particular, the cystic duct 44 is severed and the catheter 56 is inserted into the cystic duct lumen 5l such that the catheter tip 57 extends into the common bile duct lumen 52. The clip l is originally in the first configuration shown in Fig. 3 wherein the C-shaped jaws 3 and 4 are spaced on both sides.

The clip I is positioned within the tool 70 such that the outer surfaces 90 and 91 of the clip I mate with the recesses 78 and 79 of the tool. The clip I is then positioned such that the C-shaped jaws 3 and 4 are placed on opposite sides of the cystic duct 44 at a location whereat the catheter 56 is passing through the cystic duct 44 with at least several of the ridges 63 positioned beneath the clip surfaces I3 and I4. The distal ends of the tool arm 73 and 74 are urged together by manual pressure thereby urging the tool jaws 71 and 72 together and subsequently urging the clip C-shaped jaws 3 and 4 together such that jaw ends 7 and 8 touch and jaw ends II and I2 touch as seen in Fig. 4. The hinge 22 is maleable so that the arms 20 and 2I may be bent about the hinge 22 and fold the portion they are bent to without requiring additional biasing such as springs or the like. The tool 70 is then removed from the clip I as shown in Fig. 4. Dye is then injected through the catheter 56 to perform a cholangiographic process. The clip I is sized such that the surfaces I3 and I4 form tightly about the cystic duct 44 and the catheter 56 to form a tight fluid neck therebetween.

After the dye is injected, the tool jaws 71 and 72 are then placed on the clip junctures 30 and 3I and again pressure is exerted on the tool jaws 71 and 72 by manipulation of the tool arms 73 and 74. The clip junctures 30 and 3I are urged together bending the arms 20 and 2I to be more linear which rotates the clip C-shaped jaws 3 and 4 about their respective fulcrum ends II and I2 such that the distal ends 7 and 8 thereof substantially separate, thereby presenting a third or used-open configuration of the clip, as seen in Fig. 7. The clip I is then removed from the cystic duct 44 as is the catheter 56. Normally, a sealing clip (not shown) is thereafter placed on the cystic duct 44 near the common bile duct 42 and the excess cystic duct stub extending away from the clip is surgically removed.

It is to be understood that while certain forms of the present invention have been illustrated and described herein, it is not to be limited to the specific forms or arrangement of parts described and shown.

## Claims

1. A medical clip for removably clamping a living vessel (44) about a tube (56) during a medical procedure so as to hold the tube within the vessel, said clip comprising a pair of opposed jaws (3, 4) and a pair of arms (20, 21) connected at one of their ends to the jaws, characterized in that the arms are joined at their other end by a maleable hinge (22) which allows the angular spacing between the arms (20, 21) to be varied by application of pressure to the arms, and in that at least one of the arms is bent (30, 31) medially therealong outwardly with respect to the other of said arms, the ends of the jaws in touching relationship are adapted to function as a fulcrum (11, 12) so that, when further pressure is applied to said arm, the respective jaw pivots about said fulcrum, and the jaw ends (7, 8) opposite said fulcrum become spaced apart so as to allow release of the clip from the vessel.

2. A medical clip according to claim 1, characterized in that the jaws have curved opposed faces (14, 15) adapted when together to surround the vessel, each of said faces being generally C-shaped, and that the arms are connected to respective first ends of the jaws, and are joined near an end (11, 12) thereof opposite the connection to the jaws by the maleable hinge which is generally non-resilient and deformable upon application of opposed pressure to the jaws such that the angular spacing between the arms and jaws is varied by said application of pressure and the resulting configuration is retained after removal of the pressure such that, when pressure is applied to move the jaws together, said first ends of the jaws are urged into touching relationship with the jaw faces defining an opening therebetween adapted to surround the vessel while the hinge holds said jaw ends in the touching relationship.

3. A clip according to claim 1 or claim 2, characterized in that both arms (20, 21) are bent outwardly (30, 31) with respect to one another prior to use and are maleable so as to become substantially more linear by application of pressure thereto and to retain a more linear shape after pressure is removed therefrom.

4. A clip according to any one of the preceding claims, characterized in that the jaws are C-shaped in cross-section and generally encircle a cylindrical volume therebetween when abutting one another at opposite ends thereof.

5. A clip according to any one of the preceding claims, characterized in that it is shaped to snugly encircle a cystic duct with a catheter therein.

6. A tool for applying and removing a medical clip according to any one of the preceding claims, said tool including opposed jaws and pliers-like means to be operated by a user to selectively bias said jaws together and apart, characterized in that the inwardly directed surfaces (80, 81) of the mating jaws (72, 71) are contoured to provide recesses (78, 79) which can accommodate the arms (90, 91) of the clip without applying pressure thereto, a first pair of pressure applying surfaces dimensioned at least partly to surround and engage the jaws (3, 4) of said clip to move said jaws toward each other into abutting engagement and a second pair of pressure applying surfaces (85, 86) dimensioned to engage the bent arm portion (30, 31) of the clip to apply pressure at the fulcrum (11, 12) to move the clip jaws apart.

## Revendications

1. Pince médicale destinée à serrer d façon démontable un vaisseau vivant (44) autour d'un tube (56) pendant une opération médicale de façon à maintenir le tube dans le vaisseau, ladite pince comprenant une paire de mâchoires opposées (3, 4) et une paire de bras (20, 21) reliés aux mâchoires par l'une de leurs extrémités, caractérisée en ce que les bras sont réunis à leur autre extrémité par une charnière malléable (22) qui permet de modifier l'espacement angulaire entre les bras (20, 21) en appliquant une pression aux bras, et en ce qu'au moins un des bras est coudé vers l'extérieur par rapport à l'autre bras (30, 31) en un point intermédiaire de sa longueur, les extrémités des mâchoires mises en contact entre elles sont adaptées pour se comporter comme un point d'appui (11, 12) de sorte que, lorsqu'on applique davantage de pression audit bras, la mâchoire respective pivote autour dudit point d'appui et que les extrémités (7, 8) des mâchoires qui sont à l'opposé dudit point d'appui s'écartent de manière à dégager la pince du vaisseau.

2. Pince médicale selon la revendication 1, caractérisé en ce que les mâchoires possèdent des faces opposées incurvées (14, 15), qui sont adaptées pour encercler le vaisseau à elles deux, chacune desdites faces présentant la forme d'un C, et en ce que les bras sont reliés aux premières extrémités respectives des mâchoires tandis que, dans le voisinage de leur extrémité (11, 12) qui est à l'opposé de la liaison avec les mâchoires, ils sont réunis par la charnière malléable qui est sensiblement dépourvue d'élasticité et peut être déformée par l'application de pressions opposées aux mâchoires, de telle manière que l'angle formé entre les bras et les mâchoires soit modifié par ladite application de la pression et que la configuration résultante soit retenue après la suppression de la pression, de sorte que, lorsqu'une pression est appliquée pour rapprocher les mâchoires, lesdites premières extrémités des mâchoires sont sollicitées pour se placer dans des positions relatives de contact mutuel, dans lesquelles les faces des mâchoires qui définissent une ouverture entre elles sont adaptées pour encercler le vaisseau, pendant que la charnière maintient lesdites extrémités des mâchoires dans leurs positions relatives de contact mutuel.

3. Pince selon la revendication 1 ou la revendication 2, caractériée en ce qu'avant l'utilisation, les deux bras (20, 21) sont coudés vers l'extérieur (30, 31) l'un par rapport à l'autre et qu'ils sont malléables de manière à prendre une forme plus linéaire sous l'effet de l'application d'une pression et à conserver une forme plus linéaire après que la pression en a été supprimée.

4. Pince selon une quelconque des revendication précédentes, caractérisée en ce que les mâchoires sont en forme de c en section transversale et qu'elles encerclent sensiblement un volume cylindrique entre elles lorsqu'elles sont en appui l'une contre l'autre à leurs extrémités opposées.

5. Pince selon une quelconque des revendications précédentes, caractérisée en ce qu'elle est conformée de manière à encercler exactement un canal cystique avec un cathéter à l'intérieur de ce canal.

6. Outil pour la pose et l'enlèvement d'une pince médicale selon une quelconque des revendications précédentes, ledit outil comprenant des mâchoires opposées et des moyens du type pince destinés à être actionnés par un utilisateur pour resserrer ou écarter sélectivement lesdites mâchoires l'une par rapport à l'autre, caractérisé en ce que les surfaces (80, 82) des mâchoires complémentaires (72, 71) sont profilées de manière à former des évidements (78, 79) qui logent les bras (90, 91) de la pince sans leur appliquer de pression, une première paire de surfaces d'application de la pression dimensionnées pour entourer au moins partiellement et attaquer les mâchoires (3, 4) de ladite pince de manière à rapprocher lesdites mâchoires l'une de l'autre pour les mettre en appui l'une contre l'autre, et une deuxième paire de surfaces d'application de la Pression (85, 86) dimensionnées pour attaquer la portion coudée (30, 31) des bras d'une pince afin d'exercer une pression sur le point d'appui (11, 12) pour écarter les mâchoires de la pince l'une de l'autre.

## Ansprüche

1. Medizinische Klemme zum lösbaren Klemmen eines lebenden Gefäßes (44) um ein Rohr (56) während eines medizinischen Arbeitsvorganges derart, daß das Rohr in dem Gefäß gehalten wird, wobei die Klemme ein Paar einander gegenüberliegende Backen (3, 4) und ein Paar Arme (20, 21) hat, die an ihrem einen Ende mit den Backen verbunden sind, dadurch gekennzeichnet, daß die Arme an ihrem anderen Ende miteinander durch ein bleibend verformbares Gelenk (22) verbunden sind, das eine Veränderung des Winkelabstandes zwischen den Armen (20, 21) durch einen auf die Arme ausgeübten Druck gestattet, und daß mindestens einer der Arme zwischen seinen Enden gegenüber dem anderen der Arme auswärtsgebogen ist (30, 31), daß die einander berührenden Enden der Backen geeignet sind, als ein Drehpunkt (11, 12) zu wirken, so daß beim Ausüben eines weiteren Druckes auf den genannten Arm die zugeordnete Backe um den Drehpunkt verschwenkt wird und die dem Drehpunkt entgegengesetzten Backenenden (7, 8) auseinanderbewegt werden, so daß sie ein Abnehmen der Klemme von dem Gefäß ermöglichen.

2. Medizinische Klemme nach Anspruch 1, dadurch gekennzeichnet, daß die Backen einander gegenüberliegende, gekrümmte Flächen (14, 15) besitzen, die geeignet sind, das Gefäß zu umgeben, wenn sie aneinanderliegen, wobei jede dieser Flächen allgemein C-förmig ist, und daß die Arme mit einem ersten Ende je einer der Backen verbunden sind und in der Nähe ihres der Verbindung mit den Backen entgegengesetzten Endes (11, 12 miteinander durch das verformbare Gelenk miteinander verbunden sind, daß allgemein unelastisch und unter der Einwirkung einander entgegengesetzter Drücke auf die Backen derart verformbar ist, daß durch diese Druckeinwirkung der Winkelabstand zwischen den Backen und Armen und Backen verändert und die so erhaltene Form auch nach dem wegnehmen des Druckes beibehalten wird, so daß unter der Einwirkung eines Druckes, der die Backen zueinander hin zu bewegen trachtet, die ersten Enden der Backen mit den Backenflächen in Berührung gelangen, zwischen denen eine Öffnung vorhanden ist, die geeignet ist, das Gefäß zu umgeben, wenn das Gelenk die genannten Enden miteinander in Berührung hält.

3. Klemme nach Anspruch 1 oder 2, dadurch gekennzeichnet, daß vor dem Gebrauch beide Arme (20, 21) relativ zueinander auswärtsgebogen (30, 31) sind und daß sie durch einen auf sie ausgeübten Druck derart bleibend verformbar sind, daß sie beträchtlich geradliniger sind und nach der wegnahme des Druckes von ihnen eine geradlinigere Form beibehalten.

4. Klemme nach einem der vorhergehenden Ansprüche, dadurch gekennzeichnet, daß die Backen im Querschnitt C-förmig sind und wenn sie an einander entgegengesetzten Enden aneinanderliegen allgemein ein zylindrisches Volumen umgeben.

5. Klemme nach einem der vorhergehenden Ansprüche, dadurch gekennzeichnet, daß sie so geformt ist, daß sie einen Gallenausführungsgang mit darin angeordnetem Katheter satt umgeben können.

6. Werkzeug zum Anlegen und Abnehmen einer medizinischen Klemme nach einem der vorhergehenden Ansprüche, mit einander entgegengesetzten Backen und zangenartigen Mitteln, die von einem Benutzer derart betätigbar sind, daß die Backen wahlweise zueinander hin und voneinander weg belastet werden, dadurch gekennzeichnet, daß die einwärtsgekehrten Flächen (80, 81) der passend aneinanderliegenden Backen (72, 71) so profiliert sind, daß sie Vertiefungen (78, 79) bilden, die die Arme (90, 91) der Klemme aufnehmen können, ohne darauf einen Druck auszuüben, daß ein erstes Paar von Druckausübungsflächen vorgesehen sind, die so bemessen sind, daß sie die Backen (3, 4) der Klemme mindestens teilweise umgeben und derart an ihnen angreifen, daß die Backen bis zur Berührung miteinander zueinanderhin bewegt werden, und ein zweites Paar von Druckausübungsflächen (85, 86), die so bemessen sind, daß sie an dem gebogenen Armteil (30, 31) der Klemme derart angreifen können, daß auf das Gelenk (11, 12) ein Druck zum Auseinanderbewegen der Klemmbacken ausgeübt wird.

# Fig.1.

# Fig.2.

# Fig.3.

# Fig.4.

**Fig.5.**

**Fig.8.**

**Fig.6.**

**Fig.7.**